# EUROPEAN PATENT SPECIFICATION

(11) **EP 1 363 545 B1**
(45) Date of publication and mention of the grant of the patent: **07.12.2005**
(21) Application number: 02715563.9
(22) Date of filing: 25.01.2002
(51) Int. Cl.: A61B 17/72

(54) **IMPROVEMENTS IN OR RELATING TO EXPANDABLE BONE NAILS**
VERBESSERUNGEN BEI EXPANDIERBAREN KNOCHENNÄGELN
AMELIORATIONS APPORTEES A DES CLOUS A OS EXTENSIBLES

(30) Priority: 27.01.2001 GB 0102141
(43) Date of publication of application: 26.11.2003
(73) Proprietor: Davies, John Bruce Clayfield, West Lothian EH54 7BJ (GB)
(72) Inventor: Davies, John Bruce Clayfield, West Lothian EH54 7BJ (GB)
(74) Representative: Moreland, David
(86) International application number: PCT/GB2002/000311
(87) International publication number: WO 2002/058575

(56) References cited:
- EP-A- 0 970 666
- DE-A- 19 835 096
- FR-A- 2 741 256
- US-A- 3 779 239
- US-A- 4 492 226

## Description

The present invention relates to bone nails for positioning fractured bone portions relative to one another, and in particular, though not exclusively, to bone nails including expandable sections to laterally engage a cavity of each bone portion. The closest prior art is document EP-A-0970666, which defines the preamble of claim 1.

It is known that fractured bone portions can be united by the insertion of one or more bone nails (often known as intramedullary bone portion securing devices) which remain in place until the fracture is healed, and then are preferably removed though they may alternatively be renewed replaced or remain in-situ if required. Current bone nails typically have diameter selected to provide sufficient strength to the nail while remaining narrow enough to seek to limit the size of the aperture through which the bone nail is inserted; in other words the amount of bone or diameter of marrow canal which has to be removed to insert the nail. Once in position one or more sections of the nail expand to increase the diameter of the nail over each relevant section. The expanded section(s) laterally engage the wall of the bone cavity (marrow canal) to fix the nail in position. Before the nail is removed the expanded section(s) are retracted to their original diameter to aid extraction.

Intramedullary devices as referred to above may conveniently be used in the setting of bone fractures, being particularly useful in the case of long bones for example, a femur, humerus, radius or ulna. Such sites entail difficulties where the bone is required to be held in place, since these bones are usually surrounded by a considerable thickness of muscle, which spaces a rigid support of plaster of Paris from the bone by a distance which is too great to ensure that the bone is accurately aligned and firmly held. Moreover, when the arm or leg is in use, there may be rotary movement of the limb which could result in relative rotary movement between sleeve portions of the device.

Further, it is not uncommon for there to be more than one site of fracture in a bone, and particularly a long bone. Moreover, the fracture may involve splintering or fragmentation of the bone such that the overall length may be difficult to maintain whilst the bone is healing, resulting in a shortened limb.

An example of a bone nail is disclosed in US Patent No 4,453,539 to Raftopoulos et al entitled "An expandable intramedullary nail for the fixation of bone fractures". At one end of a sleeve are arranged a plurality of circumferentially spaced rectangular (or circular) openings. On rotating a screw thread along the bore of the sleeve, rectangular (or spherical) elements protrude through the sleeve to engage the bone cavity. In order to deploy the elements, an arrangement of spring clips, camming members and a pin is required. The number of parts which must be assembled to make this bone nail results in a device which is complex to construct, and hence may suffer from mechanical failure where segments fail to deploy or worse, cannot be retracted for extraction of the bone nail.

An improved bone nail is that disclosed in UK Patent No 2,268,068 to the present Applicant entitled "Devices having expansion means for securing end portions of tubular members" the content of which is included herein by reference. The disclosed device is suitable for use with a fracture of long bones, such as the humerus, femur, radius or ulna, and comprises a rod adapted to be received within an elongate cavity of the bone. The nail is provided with at least two expansion sections mounted in tandem to grip wall portions of the cavity at two locations, one on each side of the fracture site. The sections each provide a plurality of substantially rectangular slits formed in a sleeve surrounding the rod. An end surface of the sleeve abuts a flange on the rod. Tightening a nut on the rod therefore causes the slits to distort, i.e. supposedly expand outwardly. Additionally the sleeve is in the form of a number of sleeve portions which are secured together by lugs and receiving slots in each portion so as to avoid relative rotational movement between the sleeve portions and between these and the rod.

This device has an advantage that it has a simple construction. However it also has a number of disadvantages. For example, the distortion of the slits is not controlled so the expanding portions may collapse inwards rather than expanding outwards on tightening. Further, on release the sleeve portions can become disengaged from one another as the compression force is removed causing retraction and removal to fail.

It is an object of at least one embodiment of the present invention to obviate or mitigate at least one of the aforementioned disadvantages of the prior art.

According to a first aspect of the present invention, there is provided a bone portion securing device according to claim 1 adapted to be received within a bone cavity, the device including at least two expansion portions capable of being radially expanded under an applied force, each expansion portion having at least one portion, at least one characteristic of which is selected to be different to a corresponding at least one characteristic of at least one other part of the portion.

Such selection is beneficial in ensuring that the expansion portion is properly deployed, in use.

The at least one characteristic may comprise the thickness and/or width of the at least one part and the at least one other part.

Each expansion portion may comprise at least one elongate portion having a pair of elongate slots on either side thereof.

Each expansion portion may comprise a first end of the at least one elongate portion, and preferably also comprise a second end of at least one elongate portion. In such instance, the at least one other part may comprise a mid portion of the elongate portion, preferably forming a remainder of the elongate portion.

The first end and/or second end of the elongate portion may be thinner than and/or narrower than an adjacent portion of the at least one elongate portion.

Each expansion portion may comprise or further comprise a first end of at least one slot, and preferably also comprise a second end of at least one slot. In such instance, the at least one other part may comprise a mid portion of the slot forming a remainder of the slot.

The first end and/or the second end of at least one slot may be broader than an adjacent portion of the at least one slot.

According to a second aspect of the present invention, there is provided a bone securing device according to claim 1 adapted to be received within a bone cavity, the device including at least two expansion portions capable of being radially expanded under an applied force, wherein each expansion portion is shaped to elastically bow outwards when a compressive force is applied axially to the expansion member.

According to a third aspect of the present invention, there is provided a bone securing device according to claim 1 adapted to be received within a bone cavity, the device including at least two expansion portions capable of being radially expanded under an applied force, wherein each expansion portion comprises at least one longitudinal portion fixed at either end to means which engage a compression coupling, wherein the profile of the at least one longitudinal portion is narrowed at one or both ends of the at least one longitudinal portion.

Preferably, there are provided a plurality of longitudinal portions substantially equidistant spaced around a circumference of the expansion module.

Preferably, the longitudinal portion has a stepped or curved profile.

Additionally, the outer surface of the longitudinal portion may be serrated to provide grip with the inner surface of a bone.

According to a fourth aspect of the present invention there is provided a bone portion securing device according to claim 1 adapted to be received within a bone cavity, the device including at least two expansion portions capable of being radially expanded under an applied force, wherein each expansion portion includes at least one slot, the slot having at least one portion having a width greater than a width of a remainder of the at least one slot.

Preferably each expansion portion and the remainder of the slot are longitudinally displaced.

Preferably each expansion portion includes a plurality of elongate slots.

Preferably the at least one wider portion is provided at or near an end of each slot.

Advantageously each slot includes a first and second wider portions at first and second ends of the slot.

Preferably the remainder of the slot is substantially of a uniform width.

Preferably the remainder of the slot includes a first and second substantially parallel edges.

Preferably the wider portions are at least part circular in an elongate plane of the slot.

According to one embodiment of the present invention the device comprises;
at least two expansion modules, said expansion module(s) being of substantially cylindrical unitary construction including a plurality of substantially longitudinal portions which, in use, are substantially lateral to a bone wall and which bow elastically outward when a compressive force is applied axially to the expansion module;
at least one compression coupling, said compression coupling including compressive attachment means to engage the expansion module(s) in a fixed position with respect to the compression coupling and being capable of transferring a compressive force; and
at least one compression means, said compression means being arranged to transfer a force, such as a rotational force, applied to at least one portion of a surface of the compression means to a compressive force applied to the at least one compression coupling.

Preferably the plurality of longitudinal portions are substantially equidistantly spaced around a circumference of the expansion module(s).

Preferably adjacent longitudinal portions are separated from each other by an elongate slot (expansion apertures).

In longitudinal cross-section the/each slot may have one end substantially broader than the oppositely opposed end. Alternatively one or both ends may be rounded.

More preferably the cross-section of the/each lead slot is dumb-bell shaped having circular end portions and a rectangular mid section. In a preferred embodiment the end portions have a diameter which is substantially twice the width of the rectangular mid section.

The dumb-bell shape of the expansion aperture seeks to ensure that when a compressive force is applied to one or both ends of the slot in the longitudinal direction, the longitudinal portion(s) will bow outwards from the expansion module.

The longitudinal portions may also be shaped to encourage them to bow elastically outward when the compressive force is applied. The shape may provide at least one narrowed mid section. The mid section may comprise a substantially central section radially thinner than the end sections of the respective longitudinal portion. Alternatively, the longitudinal portions may be loaded by having a stepped or curved surface profile.

Preferably the compressive attachment means of the compression coupling is in the form of a ratchet. More preferably the ratchet is self locking. The compressive attachment means may engage barbs on an external surface of the expansion module(s). Preferably also the attachment means includes at least two anti-rotation grooves. The anti-rotation grooves engage a portion of an expansion module such that the expansion module cannot rotate with respect to the compression coupling. Additionally, anti-rotation grooves may be included in the expansion module.

In a preferred embodiment the combination of a self locking ratchet together with anti-rotation grooves ensures that the components of the device remain attached to each other when the nail is extracted.

Preferably the device also comprises a central connector or rod. The central connector may be a tie bar.

The expansion modules and the compression couplings may be mounted alternately onto the central connector to make up the required length of the device. Compression couplings of varying lengths can be used to allow for a desired separation between expansion modules.

The central connector may comprise a screw-threaded surface for engaging the components of the device. Alternatively the central connector may include one or more surfaces to engage at least a portion of a component mounted thereon.

Preferably the device also comprises a nose cone, positioned at a the front of the central connector or at a first expansion module, the compression means preferably being attached at a rear of the central connector or at a final compression coupling.

Preferably the nose cone has a rounded front face to provide for ease of insertion of the device into a bone cavity.

The nose cone may be attached to the central connector.

Alternatively the nose cone may be formed integrally with the central connector. Further the nose cone may include a sleeve.

Preferably also the nose cone may include rigid attachment means to engage a first expansion module in a fixed position with respect to the nose cone and consequently the central connector. Preferably the rigid attachment means is in the form of a ratchet. More preferably the ratchet is self locking. The rigid attachment may engage barbs on the external surface of the first expansion module.

The compressive attachment means and the rigid attachment means differ in that the compressive attachment means provides attachment between an expansion module and a compression coupling which, together transmit a compressive force. In contrast the rigid attachment means provides attachment between a nose cone and the first expansion module which, when a compressive force is applied to the first expansion module via the compressive means intermediary expansion module(s) and compression coupling(s), the first expansion module bears against a surface of the nose cone or a surface of the sleeve which applies the required compressive force for the longitudinal position(s) of the expansion module(s) to bow elastically outward.

Preferably the expansion module is made of a stiffly resilient plastics material. In a preferred embodiment the expansion module is made at least partly of, and preferably substantially of, titanium or optionally of a titanium alloy. Alternatively, the expansion module is made of ultra-high molecular weight polyethylene (UHMWPE). The compression coupling and the compression means may be made of a metal or metal alloy. In a preferred embodiment the compression coupling and the compression means are made of titanium alloy e.g. TiAl₆V₄.

According to a further aspect of the present invention, there is provided an expansion module for use as a portion of a bone portion securing device according to claim 1 adapted to be received within a bone cavity, the module including at least one portion capable of being radially expanded under an applied force, the at least one expansion portion having at least one portion, at least one characteristic of which is selected to be different to a corresponding at least one characteristic of at least one other portion of the portion.

According to a yet further aspect of the present invention there is provided an expansion module for use as a portion of a bone portion securing device according to claim 1 adapted to be received within a bone cavity, the module being capable of being radially expanded under an applied force, wherein the at least two expansion portions includes at least one slot the slot having at least one portion having a width greater than a width of a remainder of the at least one slot.

According to one preferred embodiment of the present invention the expansion module is of substantially cylindrical unitary construction and includes a plurality of substantially longitudinal portions which, in use, are substantially lateral to the bone wall which bow elastically outward when a compressive force is applied.

Preferably the expansion module includes coupling means for coupling the expansion module to other components of a bone portion securing device according to claim 1. Preferably the coupling means comprise barbs positioned on a surface of the expansion module such that the barbs engage in a ratchet fashion when forced against a surface of another component. The coupling means may be self locking.

More preferably the coupling means includes a portion of the expansion module which engages a portion of another component such that the expansion module and the component are held in a fixed relationship and cannot rotate with respect to each other. The portion of the expansion device may be one or more anti-rotation grooves where the component may comprise engaging lugs to the grooves or complimentary anti-rotation grooves.

Preferably the expansion module is made from a stiffly resilient plastics material such that it can elastically bow under a compressive force and return to its original shape when the compressive force is removed. In an embodiment the expansion module is made of titanium. In a further embodiment the expansion module is made from a polymer of low calcium stearate superpressed UHMWPE, with a tensile stress of 700N/mm² and a creep (over 1000 hours) of 230/250Nmm².

According to a yet further aspect of the present invention there is provided a bone portion securing device according to claim 1 comprising a plurality of parts adjacent ends of adjacent parts including one-way interengaging locking means adapted to allow engagement of the adjacent ends but to prevent disengagement thereof.

Preferably, the locking means comprise ratchet means formed on the adjacent ends.

Preferably the locking means engage one another by relative movement of the adjacent ends together.

Preferably the ratchet means comprise first and second toothed or barbed surfaces formed on an inner facing surface(s) of a first part and an outer facing surface(s) of a second part.

Advantageously the surfaces each provide a plurality of teeth or barbs.

Advantageously the surfaces are substantially planar and may be substantially parallel to a long axis of the part upon which it is formed.

Preferably there are further provided means for preventing relative rotation of the adjacent parts when assembled together.

The means to prevent relative rotation may comprise interengaging grooves and lugs carried on each of the adjacent parts.

An embodiment of the present invention will now be described by way of example with reference to the accompanying drawings in which:
Figures 1 (a), (b) and (c) show a side view, an end view and a side view to an enlarged scale of an expansion module for use in a bone portion securing device according to an embodiment of the present invention;
Figures 2 (a) to (e) show profiles of longitudinal portions of an expansion module according to embodiments of the present invention;
Figures 3 (a) to (e) show a side, a further side view, a front view, an end view and a side view to an enlarged scale of a compression coupling for use in a bone portion securing device according to the present invention;
Figures 4 (a) to (c) show a side view, a further side view and an end view of a compression nut for use in a bone portion securing device according to the present invention;
Figures 5 (a) and (b) show a cross-sectional perspective view and a cross-sectional view of a nose cone for use in a bone portion securing device according to the present invention;
Figures 6(a) and (b) show a perspective view and a side view of a first end (front) portion of a bone portion securing device according to the present invention;
Figures 7 (a) and (b) show a perspective view and a side view of a second end portion of a bone portion securing device according to the present invention;
Figure 8 shows a partial fragmentary perspective diagrammatic representation of a view of a bone portion securing device according to the present invention;
Figure 9 shows a schematic side view of a bone portion securing device according to the present invention, in use, within a bone cavity (drawn in cross-section for clarity);
Figure 10 shows (a) side views of an expansion module for use in a bone portion securing device according to a further embodiment of the present invention; and (b) a profile of a longitudinal portion of an expansion module according to the embodiments of the present invention;
Figures 11 (a) and (b) are a series of views of a yet further embodiment of an expansion module; and
Figures 12(a) and (b) are schematic representations of a bone nail according to a further embodiment of the present invention employing the expansion module of Figure 10.

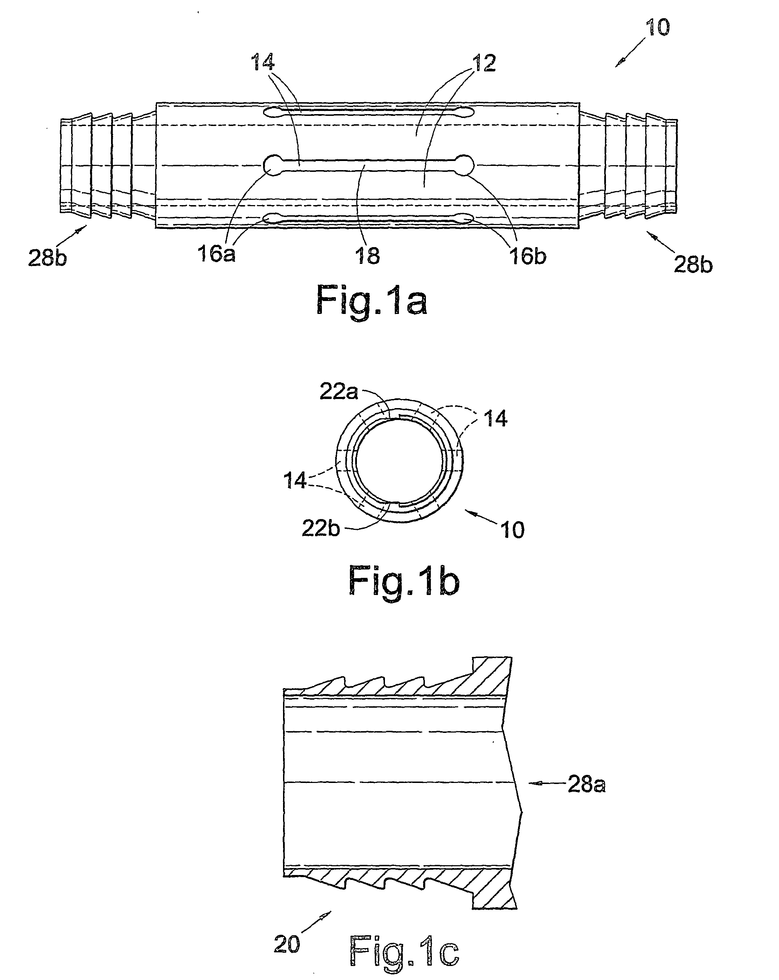

Reference is first made to Figure 1(a) of the drawings which depicts an embodiment of the present invention comprising expansion module, generally indicated by reference numeral 10. Expansion module 10 is fashioned from a single piece of tubular material, e.g. titanium. The material is selected so that it will bow under an applied compressive force without splitting, crushing or deforming. Arranged circumferentially around the expansion module 10 are six longitudinal portions 12 spaced equally apart. Between each longitudinal portion 12 is a slot in the form of an expansion aperture 14. Each expansion aperture 14 has a generally dumb-bell shape provided by forming, e.g. drilling two circular apertures 16 a, 16 b and a connecting rectangular aperture 18. The edges of the expansion apertures 14 are chamfered and the expansion apertures are drilled through the entire thickness of the wall of the expansion module 10. At each end 28a, 28b of the expansion module 10 as best illustrated in Figure 1 (c), are provided locking means comprising a set of external locking barbs 20. The barbs 20 are formed by removing an angular section of material circumferentially from the surface of the expansion module 10. Alternatively the barbs 20 could be cast or moulded with or onto the module 10. The barbs 20 are of uniform heights. In addition two small sections of each barb 20 are removed in the longitudinal direction of the expansion module 10. These small sections are formed at directly opposite sides of the expansion module 10 to form anti-rotation grooves 22a, b as shown in Figure 1 (b). The purpose of the apertures 16 a, b, barbs 20 and anti-rotation grooves 22a, b will be described hereinafter.

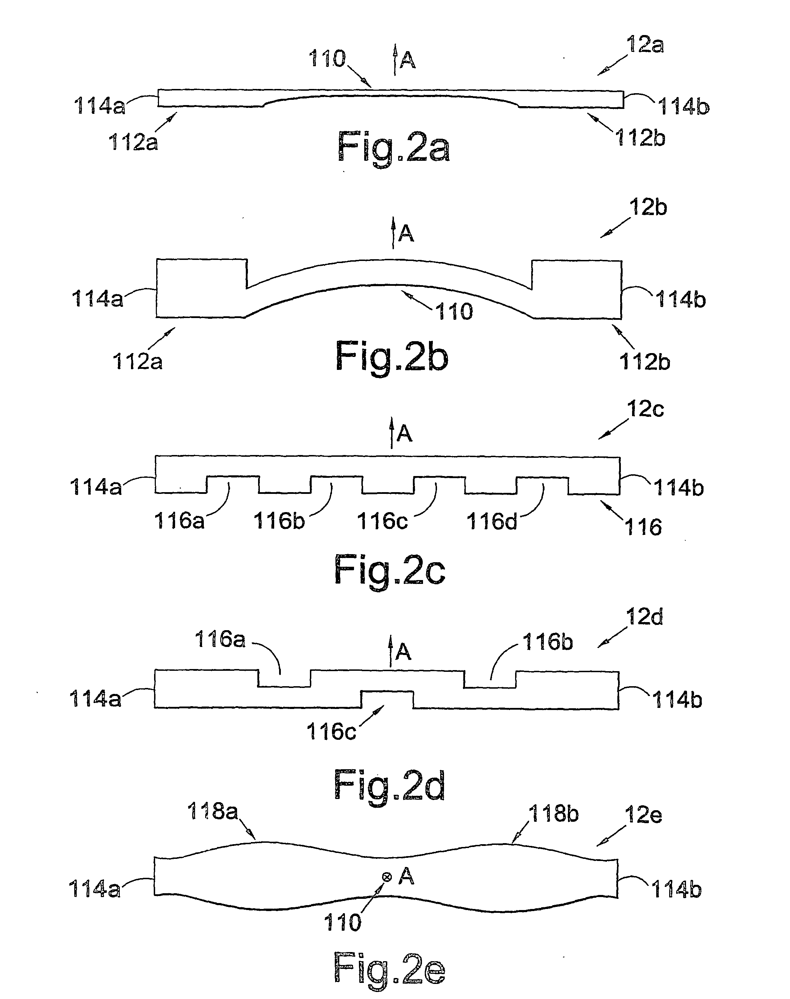

Each longitudinal portion 12 in Figure 1 is shaped to match the dumb-bell shape of the expansion apertures 14. Figures 2 (a) to (e) show alternative embodiments of longitudinal portion 12 which are loaded to ensure successful outward bowing of the longitudinal portions 12 when a force is applied. Figure 2(a) shows in radial profile a longitudinal portion 12a for an expansion module. A narrow central section 110 is weaker than wider end sections 112a, 112b so that when a compressive force is applied to ends 114a, 114b the longitudinal portion 12a will bow outwards in the direction of the arrow A causing the narrow section 110 to engage with bone.

The arrangement shown in Figure 2 (b) will provide an enhanced outward bowing effect of the longitudinal portion 12b as the central section 110 is now partly bowed when the expansion module is inserted into the bone.

Figure 2(c) shows a longitudinal portion 12c again in radial profile along the longitudinal axis of the expansion module. An inner surface 116 is stepped to provide indented portions 116a-d. This weakened inner surface will deform on compression of the ends 114a,b causing the longitudinal portion 12c to expand outwards in the direction of arrow A. In Figure 2(d) both surfaces have been stepped with indents 116a, b, c which are arranged to weaken the longitudinal portion 12d on compression causing expansion in the direction of arrow A.

Figure 2(e) shows a further embodiment of a longitudinal portion 12e viewed from above the edges of the portion 12e are curved to provide two wide sections 118a,b and a narrow central section 110 as for Figure 2(a) and (b) on compression of the ends 114a,b. The central section 110 is pushed out of the page in the direction of A.

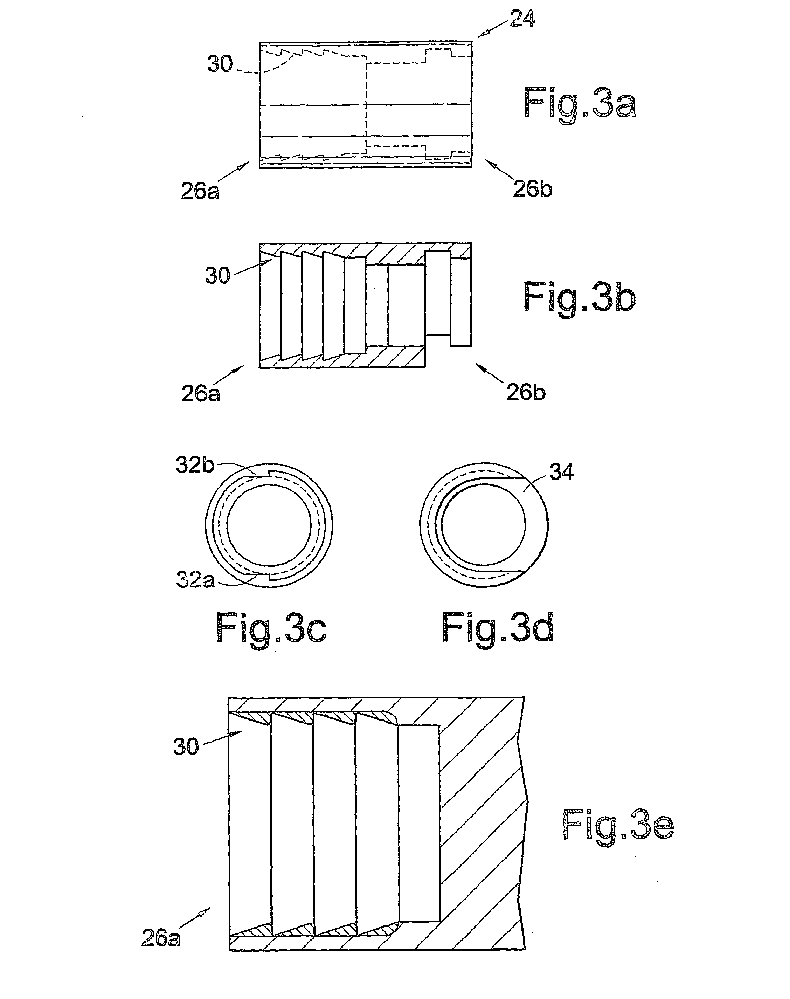

Reference is now made to Figure 3 (a) of the drawings which depicts a compression coupling, generally indicated by reference numeral 24. A first end 26a provides a receiving ratchet of dimensions to engage an end 28a, 28b of an expansion module 10, as shown in Figure 3 (e) Inwardly opposed barbs 30 also include matching anti-rotation grooves 32a, 32b to the grooves 22a, 22b of the expansion module 10. The anti-rotation grooves 32a, 32b are shown in Figure 3 (c) . At a second end 26b, figures 3(b) and 3(d) is a transverse slot 34. The slot 34 provides for the engagement of the coupling 24 to a compression nut.

In a further embodiment of the present invention the coupling 24 has substantially identical ends both as described for the first end 26a above.

The coupling 24 may be constructed of TiAl₆, biocompatible non-corrosive alloy, which is strong enough to transmit a compressive force. The length of the coupling 24 is dependent on the separation required between expansion modules 10.

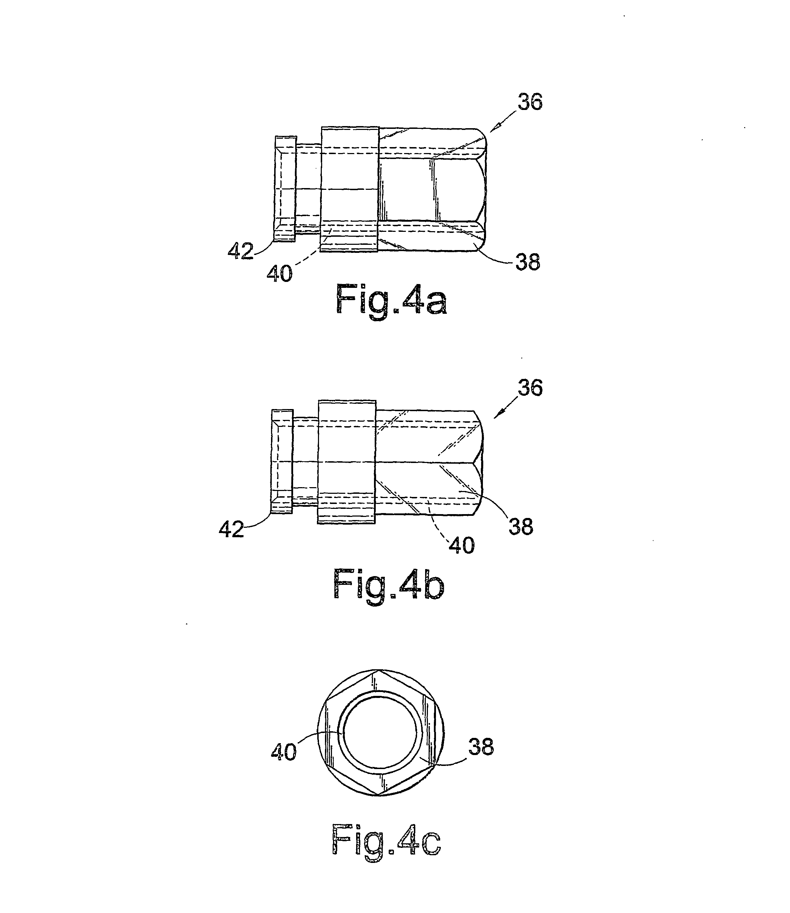

A coupling means comprising a nut 36 is illustrated in Figure 4. The nut 36 is conveniently made of the same material as the compression coupling 24. The nut 36 comprises a hex screw head 38 which can be turned by a spanner or socket set, Internal to the nut 36 is a screw-threaded bore 40 which extends the length of the nut 36. One end 42 of the nut 40 is configured to mate with the transverse slot 34 of the compression coupling 24 described above.

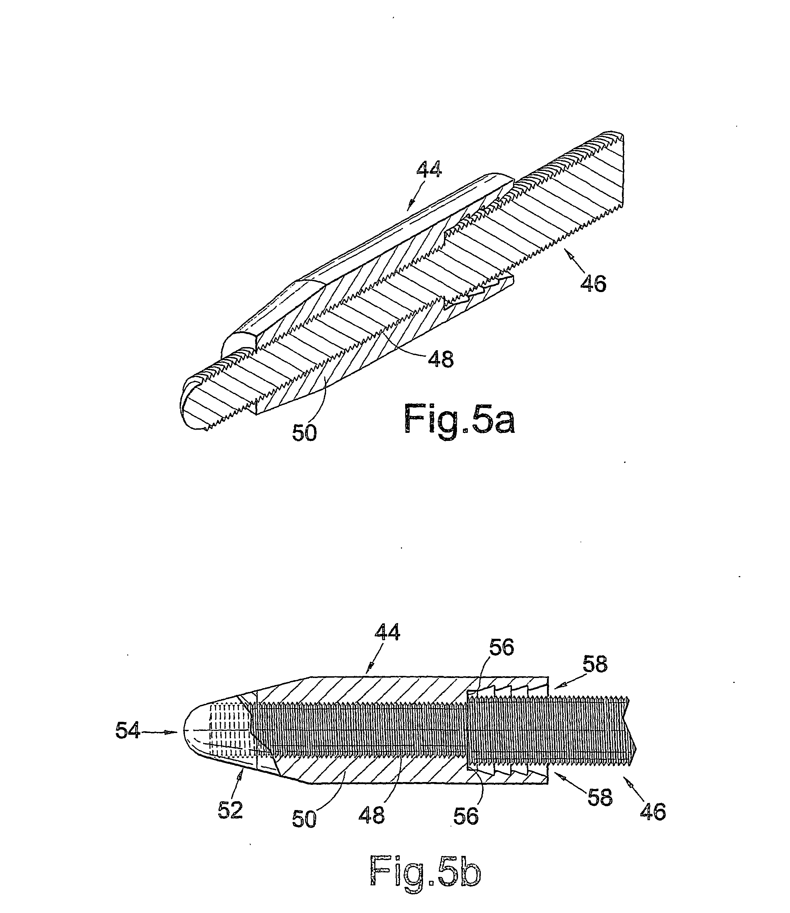

A further component of an intermedullary bone nail is shown in Figure 5 and is generally referred to as a nose cone 44. A central tie bar 48 is a screw threaded rod running the length of the bone nail. An end section 46 of the bar 48 is threaded at a smaller size and fits into the nose cone body 50 which is bored and tapped. On assembling the nose cone 44, the end section 46 is welded or otherwise fixed into the nose cone body 50 and a TIG welded chamber 52 is provided to give a rounded front face 54 to the nose cone 44. A circumferential portion 58 of the nose cone 44 is formed to receive an end 28 of an expansion module by the same arrangement as described for the first end 26a of the compression coupling 24. At a front end of the circumferential portion 58 are surfaces which represent the compression face 56 for the bone nail.

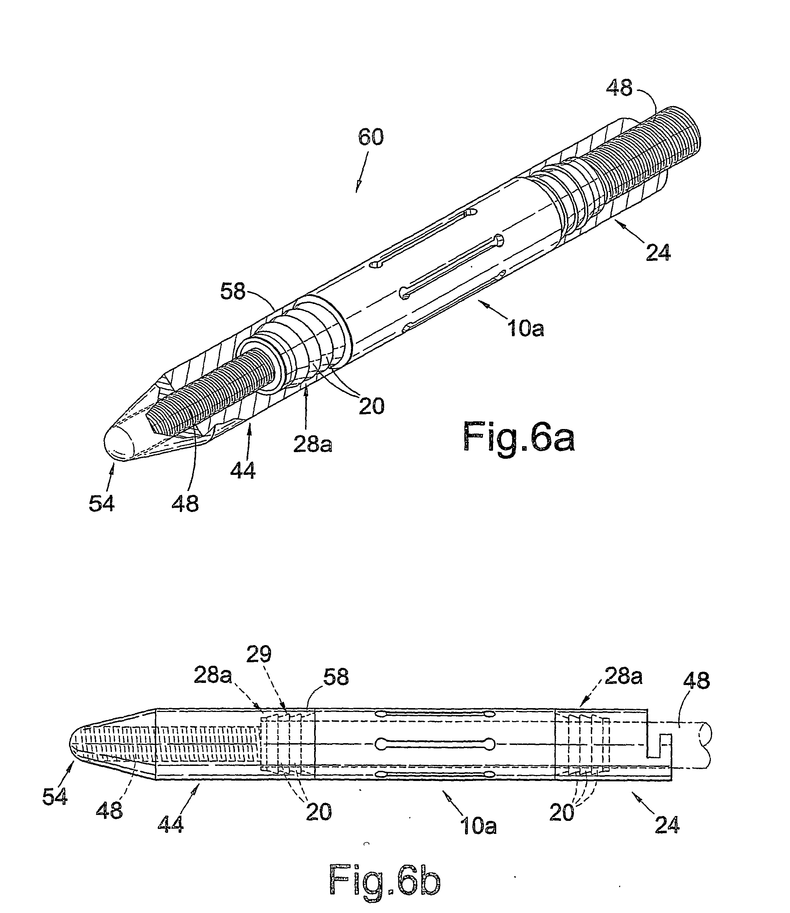

The construction of an embodiment of an intermedullary 25 bone portion securing device 61 will now be described making reference to Figures 6, 7 and 8.

A first expansion module 10a is inserted over the tie bar 48 and the barbs 20 at a first end 28a of the expansion module 10a engage the circumferential portion 58 of the nose cone 44 in a self locking ratchet mechanism.

In addition complimentary anti-rotation grooves 32a, 32b interlock to prevent rotational movement of the expansion module 10a with respect to the nose cone 44. The barbs 20 of the second end 28b of the expansion module 10a mate with a compression coupling 24 which is mounted on the tie bar 48. Mating is by the self locking ratchet mechanism 29 and anti-rotation grooves 32a, 32b described hereinbefore. A joint assembly of the front 60 of a bone portion securing device 61 is shown in Figure 6.

Depending on the separation required between the two sets of longitudinal portions on adjacent expansion modules 10, one or more compression couplings 24 are mounted on the tie bar 48. Alternatively a single compression coupling 24 of the desired length is used. Any number of expansion modules 10 may be mounted on the tie bar 48 and it is optional as to whether compression couplings 24 are inserted between each expansion module 10.

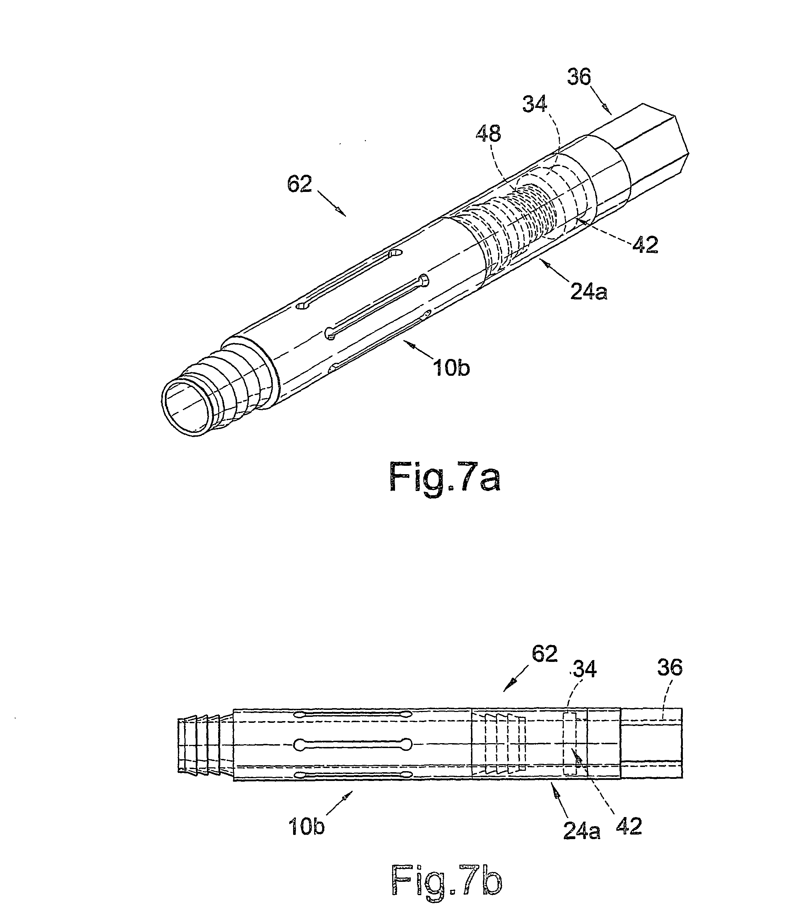

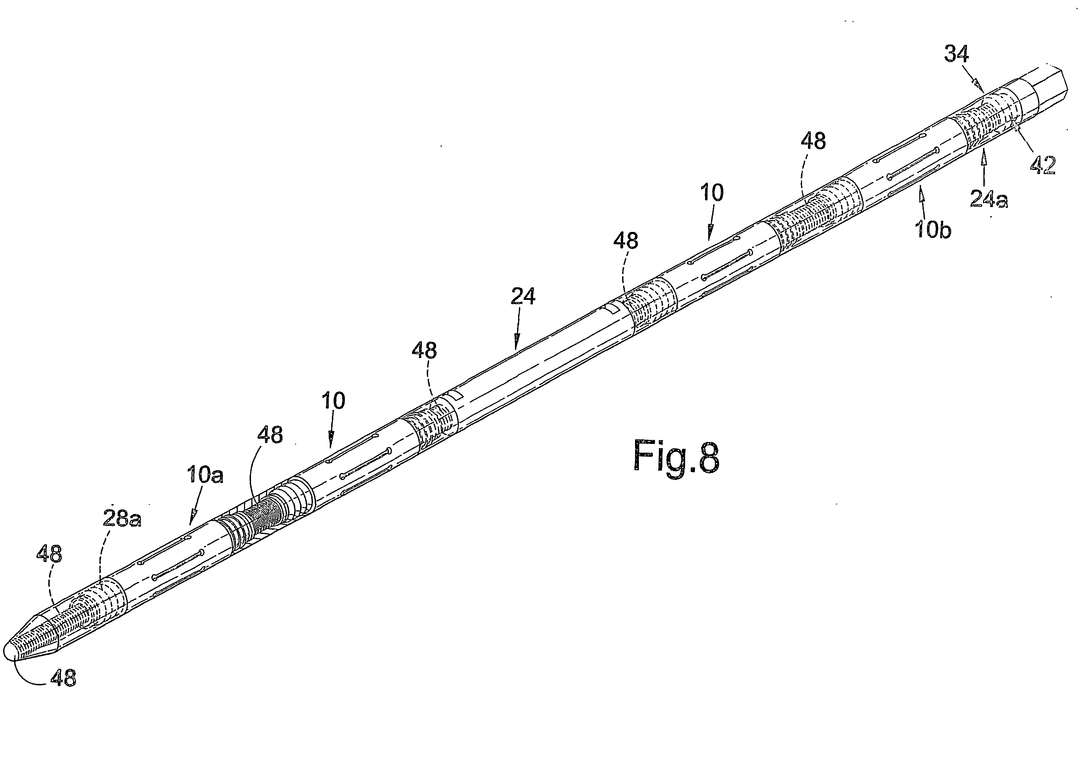

When the desired number of expansion modules 10 have been inserted onto the tie bar 48 an end joint assembly 62 is constructed as shown in Figure 7. Once the final expansion module 10b is mounted on the tie bar 48 the bone nail 61 is completed with a final compression coupling 24a and the compression nut 36. The end 42 of the compression nut 36 is slid into the transverse slot 34 of the compression coupling 24a. When engaged together the coupling 24a is mounted on the tie bar 48 and the nut 36 is screwed onto the end of the tie bar 48 to hold all components of the bone nail 61 in place. The compressive coupling 24a is held to the final expansion module 10b by a self locking ratchet mechanism and anti-rotation grooves 32a, 32b as described before. An exploded view of a complete bone portion securing device 61 is illustrated in Figure 8.

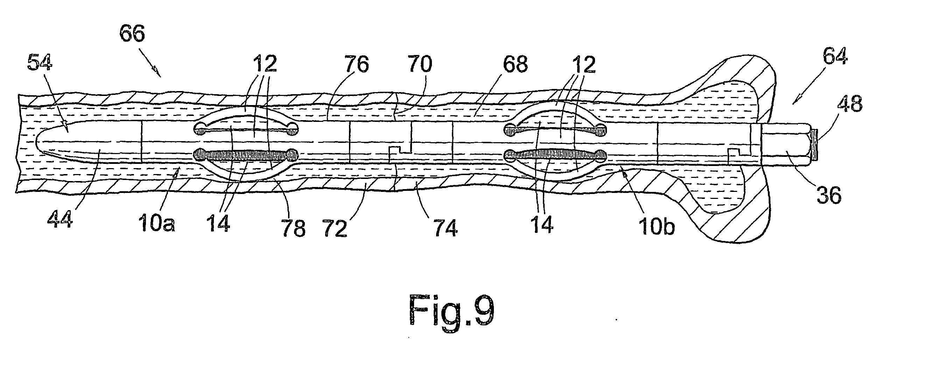

Reference is now made to Figure 9 of the drawings which illustrates an intermedullary bone portion securing device, generally indicated by reference numeral 10a, in use within a fractured bone generally indicated by reference numeral 66. The bone 66 has been drawn in cross-section to allow for clarity of view of the device 10a. The device 10a is shown received in the narrow channel or cavity 68 of the bone 66, which in the present example is a mammalian humerus. The bone 66 is shown with a fracture at 70 which requires bone portions 72 and 74 to be reunited.

The surgeon or veterinary practitioner will have pre-drilled a bore 76 through the bone cavity 68 to receive the device 64. This bone will be drilled at the elbow. With the compression nut 36 in an untightened position with all the components mounted on the tie bar 48, the device 61 is pushed into the bone 76. The front face 54 of the device 64 is rounded to reduce friction and aid insertion. The device 64 is positioned so that an expansion module 10a, 10b lies on either side of the fracture 70. When the correct positioning is obtained the compression nut 36 is tightened using a spanner or socket wrench. On tightening the nut 36 the components are forced against each other towards the nose cone 44. The front end 28a of the first expansion module 10a bears against the compression face 56 of the nose cone 44 and applies a compression force. This is shown and described with reference to Figure 5. The force is transmitted through the components.

When each expansion module 10a, 10b is subjected to the axial compressive force, the expansion apertures 14 begin to widen and the longitudinal portions 12 bow outwards elastically. As the force is increased the length of each expansion module 10a, b decreases and the effective diameter of each expansion module 10a, 10b increases. The longitudinal portions 12 bow until they contact the bone cavity wall 76. The shape of the expansion apertures 14 ensure that the longitudinal portions 12 bow outwards from the tie bar 48 and central axis of the device 64. Once expanded, the device 61 is held in position by the contact between the cavity wall 78 and the longitudinal portions 12. The expansion modules 10a, 10b remain expanded as long as the compression nut 36 remains tightened.

Once the fracture 70 has healed or if the device 61 requires to be removed, the compression nut 36 is loosened by reversing the above procedure. This releases the compressive force and the expansion modules 10a, 10b lengthen as the longitudinal portions 12 disengage from the cavity wall 78 and return to their original positions. The device 61 is then extracted from the healed bone by pulling the device 61 from the bone 76. The incorporation of anti-rotation grooves and ratchet fittings between the components of the device 61 seeks to ensure that the components do not become detached from the device 61 during the extraction process.

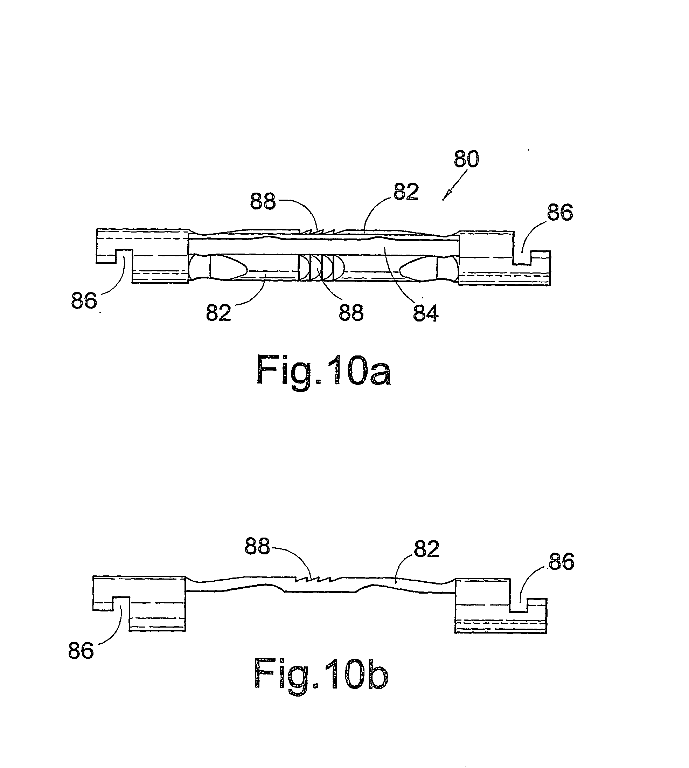

Figure 10(a) depicts a further embodiment of the present invention comprising exapnsion module 80. Arranged circumferentially around the expansion module 10c are three longitudinal portions 82. Between each longitudinal portion 82 is a slot in the form of an expansion aperture 84. Locking means 86 are provided at each end of the expansion module.

The longitudinal portions 82 are loaded to ensure successful outward bowing of the portions when a force is applied. Figure 10(b) illustrates in detail such a longitudinal portion in profile. An inner and outer surface are curved to provide a weakened inner surface which will deform outwards on compression of the ends. A serrated area 88 of the outer surface of the longitudinal portion 82 provides grip with the inner surface of the bone (not shown).

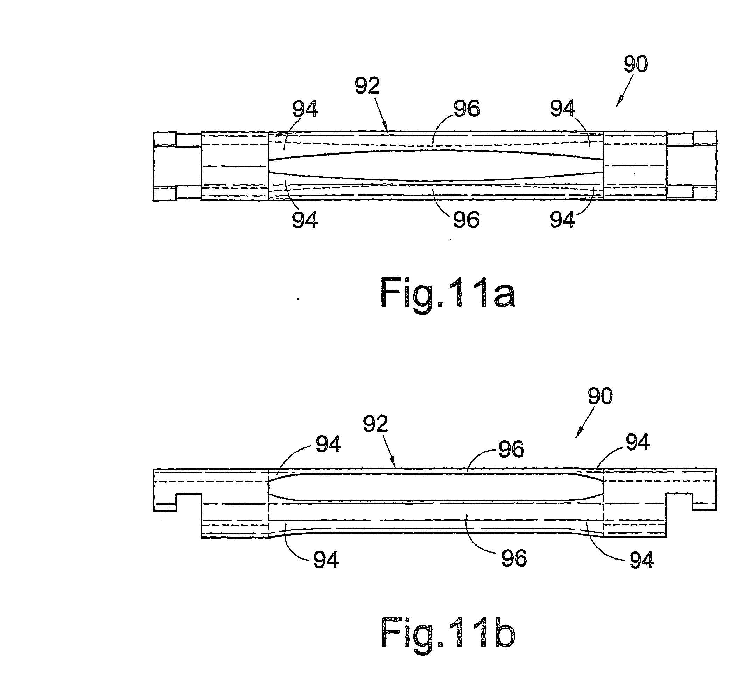

Figures 11 (a) and (b) are a series of views of a yet further embodiment of an expansion module 90. In this embodiment 90, the character that is varied is the thickness. End portions 94 of the strip 92 are broader as compared to the mid portions 96 of said strip 92.

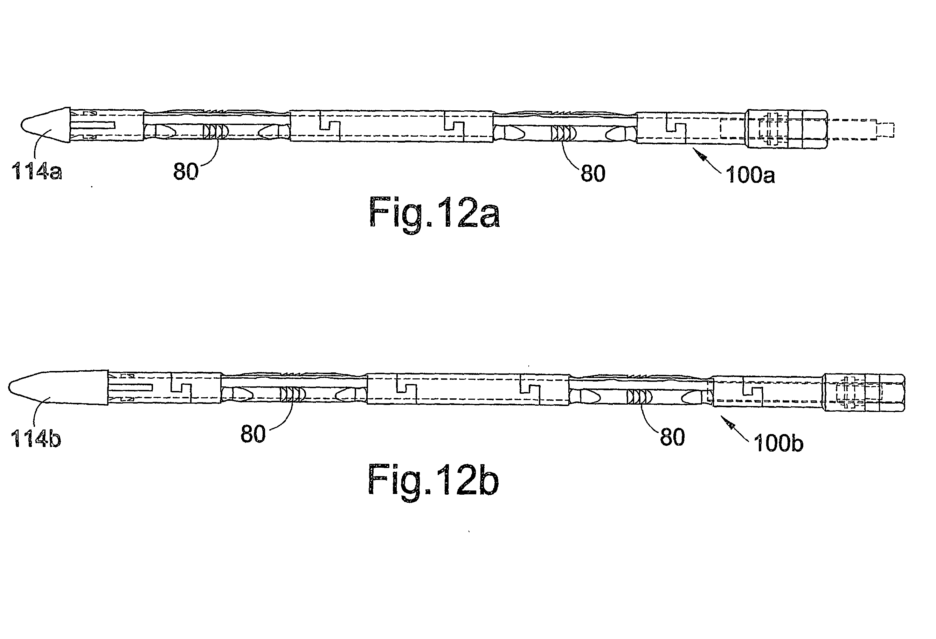

Figures 12(a) and (b) are schematic representations of a bone nail 100 according to a further embodiment of the present invention employing the expansion module 80 of Figure 10. The assembly of the bone nail 100 includes couplings 112 to couple together two expansion modules 80. The assembly also includes nose cone 114a in Figure 12(a) and a modified nose cone 114b in Figure 12(b).

A principal advantage of the present invention is that it provides a modular self locking IC humeral nail, In particular the bone portion securing device is designed to be fail safe in that, under compression, an expansion module of the device will always expand outwards to contact the cavity walls. In addition the device is designed so that it will not come apart when extracted from a bone.

It will be appreciated by one skilled in the art that various modifications may be made to the embodiments described hereinbefore without departing from the scope of the invention.

In particular, it will be appreciated that there may well be gaps between bone edges at any of the fractures but this is in no way detrimental to the operation of the securing device of the invention. Such gaps will in any case tend to fill in as the bone heals.

It will also be appreciated that the use of a device according to the invention may be controlled to an appreciable degree by the manner in which the apertures of the expansion module is formed. The extent of the deformation brought about by the application of compressive force in a longitudinal direction can be controlled by selection from a number of variables, e.g. number of apertures in each annular group, thickness of expansion module wall, choice of material for the expansion module, width and/or length of apertures, width of position intervening between the apertures and so on. Moreover, the deformation characteristics of the expansion modules may be selected to vary between two or more area of the bone, so that selection expansion, sequentially arranged if desired, can be achieved.

It will also be understood that the cross-section of the expansion modules, longitudinal portions and couplings, although described as above as being conveniently circular, may be oval, triangular or any convenient cross-section as desired.

In addition, as briefly referred to above, there is the facility now provided for adjustment of the device during the insertion thereof if required.

It is also possible to select the material of the expansion modules and couplings so that they are degradable in time and does not require to be removed once the bone has healed. The rod means may be withdrawn from the bone through the access point, e.g. the elbow, at which actuation of the expansion devices was brought about.

## Claims

1. A bone portion securing device (10; 10a,10b; 80; 90) adapted to be received within a bone cavity (68), the device including at least two expansion portions (12; 82; 92) capable of being radially expanded under an applied force, each expansion portion having at least one portion and at least one other portion, at least one characteristic of the at least one portion being selected to be different to a corresponding at least one characteristic of the at least one other portion , and wherein a slot (14; 84; 94) is provided between adjacent expansion portions, **characterised by** each side of each slot being chamfered with respect to a radial direction passing through the respective side.

2. A bone portion securing device as claimed in claim 1, wherein sides of a given slot are substantially planarly parallel to one another, and parallel to a radial direction passing between the sides of the given slot.

3. A bone portion securing device as claimed in claim 2, wherein the device has a substantially circular cross-section.

4. A bone portion securing device as claimed in any of claims 1 to 4, wherein the at least one characteristic comprises a thickness and/or width of the at least one part and the at least one other part.

5. A bone portion securing device as claimed in any of claims 1 to 4, wherein the expansion portion comprises at least one elongate portion having a pair of elongate slots on either side thereof.

6. A bone portion securing device according to any of claims 1 to 5, wherein the at least one portion comprises a first end of the at least one elongate portion and a second end of the at least one elongate portion.

7. A bone portion securing device as claimed in claim 6, wherein the at least one other part comprises a mid portion of the elongate portion forming a remainder of the elongate portion.

8. A bone portion securing device as claimed in either of claims 6 or 7, wherein the first end and/or second end of the elongate portion is thinner or thicker and/or narrower or broader than an adjacent portion of the at least one elongate portion.

9. A bone portion securing device as claimed in any of claims 1 to 3, wherein the at least one portion comprises or further comprises a first end (16a) of at least one slot and a second end (16b) of at least one slot.

10. A bone portion securing device as claimed in claim 9, wherein the at least one other part comprises a mid portion (18) of the slot forming a remainder of the slot.

11. A bone portion securing device as claimed in claim 1, wherein the first end and/or the second end of the at least one slot is broader than an adjacent portion of the at least one slot.

12. A bone securing device as claimed in any of claims 1 to 3, wherein each expansion portion is shaped to elastically bow outwards when a compressive force is applied axially to the expansion portions.

13. A bone securing device as claimed in any of claims 1 to 3, wherein each expansion portion comprises at least one longitudinal portion fixed at either end to means which engage a compression coupling (24), wherein a profile of the at least one longitudinal portion is narrowed at one or both ends of the at least one longitudinal portion.

14. A bone portion securing device as claimed in claim 13, wherein there are provided a plurality of longitudinal portions substantially equidistantly spaced around a circumference of the expansion module.

15. A bone portion securing device as claimed in either of claims 13 or 14, wherein each longitudinal portion has a stepped or curved profile.

16. A bone portion securing device as claimed in any of claims 13 to 15, wherein the outer surface of each longitudinal portion is serrated (88) to provide grip with an inner surface of a bone.

17. A bone portion securing device as claimed in any of claims 1 to 3, wherein each expansion portion includes a slot on either longitudinal side thereof, each slot having at least one portion having a width greater than a width of a remainder of the at least one slot.

18. A bone portion securing device as claimed in claim 17, wherein the at least one portion of each slot and the remainder of each slot are longitudinally displaced.

19. A bone portion securing device as claimed in either of claims 17 or 18, wherein the/each expansion portion includes a plurality of elongate slots.

20. A bone portion securing device as claimed in any of claims 17 to 19, wherein each slot includes first and second wider portions at first and second ends of the slot.

21. A bone portion securing device as claimed in claim 20, wherein a remainder of the slot is substantially uniform width.

22. A bone portion securing device as claimed in claim 1, wherein the device comprises a single piece of tubular material.

23. A bone portion securing device as claimed in claim 1, wherein at least part of an outer surface of each expansion portion is concave and at least part of an inner surface of each expansion portion is concave.

24. A bone portion securing apparatus comprising:
at least two expansion modules, each expansion module comprising a bone portion securing device (10; 10a; 10b; 80;90) according to any of claims 1 to 23, each device being of substantially cylindrical unitary construction, each expansion portion comprising a plurality of substantially longitudinal portions which, in use, are substantially lateral to a bone wall and which bow elastically outward when a compressive force is applied axially to the respective expansion module;
at least one compression coupling (24), the compression coupling including compressive attachment means to engage the expansion modules in a fixed position with respect to the compression coupling and being capable of transferring a compressive force; and
at least one compression means, the compression means being arranged to transfer a force, such as a rotational force, applied to at least one portion of a surface of the compression means, in use, to a compressive force applied to the at least one compression coupling.

25. A bone portion securing apparatus as claimed in claim 24, wherein the plurality of longitudinal portions are substantially equidistantly spaced around a circumference of each expansion module and are separated from each other by a slot which comprises an elongate slot.

26. A bone portion securing apparatus according to either of claims 24 or 25, wherein one or both ends of each slot are rounded in longitudinal cross-section.

27. A bone portion securing apparatus as claimed in either of claims 24 or 25, wherein the longitudinal portions are loaded by having a stepped or curved surface profile.

28. A bone portion securing apparatus as claimed in claims 24, wherein the compressive attachment means of the compression coupling is in the form of a self-locking ratchet.

29. A bone portion securing apparatus as claimed in claim 24, wherein the attachment means includes at least two anti-rotation grooves (32a,32b) which engage a portion of an expansion module such that the expansion module cannot rotate with respect to the compression coupling.

30. A bone portion securing device as claimed in any of claims 1 to 23 or a bone portion securing apparatus as claimed in any of claims 25 to 29, wherein the device or expansion module is made of a stiffly resilient plastics material, titanium or titanium alloy.

31. A bone portion securing apparatus as claimed in claim 24, wherein the apparatus is adapted such that, in use, one at least one expansion module lies on one side of a fracture in a bone and one other at least one expansion module lies on another side of the fracture.

32. A bone portion securing apparatus as claimed in claim 24, wherein the expansion modules are provided on a tie-bar.

33. A bone portion securing apparatus as claimed in claim 32, wherein adjacent expansion modules are mated by means of a compression coupling (24).

## Patentansprüche

1. Knochenbefestigungsvorrichtung (10; 10a, 10b; 80; 90), die so gestaltet ist, dass sie in einem Knochenhohlraum (68) aufgenommen wird, wobei die Vorrichtung wenigstens zwei Expansionsabschnitte (12; 82; 92) beinhaltet, die unter einer aufgebrachten Kraft radial expandieren können, wobei jeder Expansionsabschnitt wenigstens einen Abschnitt und wenigstens einen weiteren Abschnitt aufweist, wobei wenigstens eine Charakteristik des wenigstens einen Abschnitts so gewählt wird, dass sie sich von einer entsprechenden wenigstens einen Charakteristik des wenigstens einen anderen Abschnitts unterscheidet, und wobei ein Schlitz (14; 84; 94) zwischen benachbarten Expansionsabschnitten vorgesehen ist, **dadurch gekennzeichnet, dass** jede Seite jedes Schlitzes in Bezug auf eine durch die jeweilige Seite passierende radiale Richtung abgefast ist.

2. Knochenbefestigungsvorrichtung nach Anspruch 1, wobei Seiten eines gegebenen Schlitzes im Wesentlichen flachparallel zueinander und parallel zu einer zwischen den Seiten des gegebenen Schlitzes passierenden radialen Richtung sind.

3. Knochenbefestigungsvorrichtung nach Anspruch 2, wobei die Vorrichtung einen im Wesentlichen kreisförmigen Querschnitt hat.

4. Knochenbefestigungsvorrichtung nach einem der Ansprüche 1 bis 4, wobei die wenigstens eine Charakteristik die Dicke und/oder Breite des wenigstens einen Teils und des wenigstens einen anderen Teils umfasst.

5. Knochenbefestigungsvomchtnng nach einem der Ansprüche 1 bis 4, wobei der Expansionsabschnitt wenigstens einen länglichen Abschnitt mit einem Paar länglichen Schlitzen auf beiden Seiten davon umfasst.

6. Knochenbefestigungsvorrichtung nach einem der Ansprüche 1 bis 5, wobei der wenigstens eine Abschnitt ein erstes Ende des wenigstens einen länglichen Abschnitts und ein zweites Ende des wenigstens einen länglichen Abschnitts umfasst.

7. Knochenbefestigungsvorrichtung nach Anspruch 6, wobei der wenigstens eine andere Teil einen Mittelabschnitt des länglichen Abschnitts umfasst, der den Rest des länglichen Abschnitts bildet.

8. Knochenbefestigungsvorrichtung nach Anspruch 6 oder 7, wobei das erste und/oder das zweite Ende des länglichen Abschnitts dünner oder dicker und/oder schmäler oder breiter ist als der benachbarte Abschnitt des wenigstens einen länglichen Abschnitts.

9. Knochenbefestigungsvorrichtung nach einem der Ansprüche 1 bis 3, wobei der wenigstens eine Abschnitt ein erstes Ende (16a) des wenigstens einen Schlitzes und ein zweites Ende (16b) des wenigstens einen Schlitzes umfasst oder ferner umfasst.

10. Knochenbefestigungsvorrichtung nach Anspruch 9, wobei der wenigstens andere Teil einen Mittelabschnitt (18) des den Rest des Schlitzes bildenden Schlitzes umfasst.

11. Knochenbefestigungsvorrichtung nach Anspruch 1, wobei das erste Ende und/oder das zweite Ende des wenigstens einen Schlitzes breiter ist als ein benachbarter Abschnitt des wenigstens einen Schlitzes.

12. Knochenbefestigungsvorrichtung nach einem der Ansprüche 1 bis 3, wobei jeder Expansionsabschnitt so gestaltet ist, dass er sich elastisch auswärts biegt, wenn eine Kompressionskraft axial auf die Expansionsabschnitte aufgebracht wird.

13. Knochenbefestigungsvorrichtung nach einem der Ansprüche 1 bis 3, wobei jeder Expansionsabschnitt wenigstens einen Längsabschnitt umfasst, der an einem Ende mit Mitteln befestigt ist, die in eine Kompressionskupplung (24) eingreifen, wobei ein Profil des wenigstens einen Längsabschnitts an einem oder an beiden Enden des wenigstens einen Längsabschnitts schmäler ist.

14. Knochenbefestigungsvorrichtung nach Anspruch 13, wobei eine Mehrzahl von Längsabschnitten vorgesehen ist, die im Wesentlichen gleichmäßig um einen Umfang des Expansionsmoduls beabstandet sind.

15. Knochenbefestigungsvorrichtung nach Anspruch 13 oder 14, wobei jeder Längsabschnitt ein gestuftes oder gekrümmtes Profil hat.

16. Knochenbefestigungsvorrichtung nach einem der Ansprüche 13 bis 15, wobei die Außenfläche jedes Längsabschnitts gezackt (88) ist, um einen Eingriff in eine Innenfläche eines Knochens zu ermöglichen.

17. Knochenbefestigungsvorrichtung nach einem der Ansprüche 1 bis 3, wobei jeder Expansionsabschnitt einen Schlitz auf seinen beiden Längsseiten beinhaltet, wobei jeder Schlitz wenigstens einen Abschnitt mit einer Breite hat, die größer ist als die Breite des Rests des wenigstens einen Schlitzes.

18. Knochenbefestigungsvorrichtung nach Anspruch 17, wobei der wenigstens eine Abschnitt jedes Schlitzes und der Rest jedes Schlitzes longitudinal verschoben sind.

19. Knochenbefestigungsvorrichtung nach Anspruch 17 oder 18, wobei der/jeder Expansionsabschnitt eine Mehrzahl von länglichen Schlitzen beinhaltet.

20. Knochenbefestigungsvorrichtung nach einem der Ansprüche 17 bis 19, wobei jeder Schlitz erste und zweite breitere Abschnitte am ersten und am zweiten Ende des Schlitzes aufweist.

21. Knochenbefestigungsvorrichtung nach Anspruch 20, wobei der Rest des Schlitzes eine im Wesentlichen gleichförmige Breite hat.

22. Knochenbefestigungsvorrichtung nach Anspruch 1, wobei die Vorrichtung ein einzelnes Stück aus röhrenförmigem Material umfasst.

23. Knochenbefestigungsvorrichtung nach Anspruch 1, wobei wenigstens ein Teil einer Außenfläche jedes Expansionsabschnitts konkav und wenigstens ein Teil einer Innenfläche jedes Expansionsabschnitts konkav ist.

24. Knochenbefestigungsvorrichtung, die Folgendes umfasst:
wenigstens zwei Expansionsmodule, wobei jedes Expansionsmodul eine Knochenbefestigungsvorrichtung (10; 10a; 10b; 80; 90) nach einem der Ansprüche 1 bis 23 umfasst, wobei jede Vorrichtung im Wesentlichen einheitlich zylindrisch aufgebaut ist, wobei jeder Expansionsabschnitt eine Mehrzahl von im Wesentlichen longitudinalen Abschnitten umfasst, die beim Gebrauch im Wesentlichen lateral zu einer Knochenwand sind und die sich elastisch auswärts biegen, wenn eine Kompressionskraft axial auf das jeweilige Expansionsmodul aufgebracht wird;
wenigstens eine Kompressionskupplung (24), wobei die Kompressionskupplung Kompressionsbefestigungsmittel aufweist, um in die Expansionsmodule in einer festen Position mit Bezug auf die Kompressionskupplung einzugreifen und um eine Kompressionskraft übertragen zu können; und
wenigstens ein Kompressionsmittel, wobei das Kompressionsmittel die Aufgabe hat, eine Kraft, wie z.B. eine Drehkraft, die auf wenigstens einen Abschnitt einer Oberfläche des Kompressionsmittels beim Gebrauch aufgebracht wird, in eine Kompressionskraft umzuwandeln, die auf die wenigstens eine Kompressionskupplung aufgebracht wird.

25. Knochenbefestigungsvorrichtung nach Anspruch 24, wobei die Mehrzahl der Längsabschnitte im Wesentlichen gleichmäßig um einen Umfang jedes Expansionsmoduls herum beabstandet und durch einen Schlitz voneinander getrennt sind, der einen länglichen Schlitz umfasst.

26. Knochenbefestigungsvorrichtung nach Anspruch 24 oder 25, wobei ein oder beide Enden jedes Schlitzes im longitudinalen Querschnitt abgerundet ist/sind.

27. Knochenbefestigungsvorrichtung nach Anspruch 24 oder 25, wobei die Längsabschnitte beladen sind, indem sie ein gestuftes oder gekrümmtes Oberflächenprofil haben.

28. Knochenbefestigungsvorrichtung nach Anspruch 24, wobei das Kompressionsbefestigungsmittel der Kompressionskupplung die Form einer Selbstsperrungsklinke hat.

29. Knochenbefestigungsvorrichtung nach Anspruch 24, wobei das Befestigungsmittel wenigstens zwei Rotationssicherungskerben (32a, 32b) aufweist, die in einen Abschnitt eines Expansionsmoduls eingreifen, so dass das Expansionsmodul nicht in Bezug auf die Kompressionskupplung rotieren kann.

30. Knochenbefestigungsvorrichtung nach einem der Ansprüche 1 bis 23 oder Knochenbefestigungsvorrichtung nach einem der Ansprüche 25 bis 29, wobei die Vorrichtung oder das Expansionsmodul aus einem steif elastischen Kunststoffmaterial, aus Titan oder aus einer Titanlegierung gefertigt ist.

31. Knochenbefestigungsvorrichtung nach Anspruch 24, wobei die Vorrichtung so gestaltet ist, dass beim Gebrauch wenigstens ein Expansionsmodul auf einer Seite einer Fraktur in einem Knochen und wenigstens ein weiteres Expansionsmodul auf einer anderen Seite der Fraktur liegt.

32. Knochenbefestigungsvorrichtung nach Anspruch 24, wobei die Expansionsmodule auf einem Zugstab vorgesehen sind.

33. Knochenbefestigungsvorrichtung nach Anspruch 32, wobei benachbarte Expansionsmodule mit einer Kompressionskupplung (24) zusammengesteckt werden.

## Revendications

1. Dispositif de fixation de partie d'os (10; 10a, 10b; 80; 90) adapté pour être reçu à l'intérieur d'une cavité d'os (68), le dispositif comprenant au moins deux parties d'expansion (12; 82; 92) capables d'être étendues radialement sous une force appliquée, chaque partie d'expansion possédant au moins une partie et au moins une autre partie, au moins une caractéristique de la au moins une partie étant sélectionnée pour être différente de la au moins une caractéristique correspondante de la au moins une autre partie, et dans lequel une encoche (14; 84; 94) est prévue entre les parties d'expansion adjacentes, **caractérisé par le fait que** chaque côté de chaque encoche est chanfreiné par rapport à la direction radiale passant à travers le côté respectif.

2. Dispositif de fixation de partie d'os comme revendiqué dans la revendication 1, dans lequel les côtés d'une encoche donnée sont de manière essentiellement planaire parallèles l'un à l'autre, et parallèles à la direction radiale passant entre les côtés de l'encoche donnée.

3. Dispositif de fixation de partie d'os comme revendiqué dans la revendication 2, dans lequel le dispositif possède une section transversale essentiellement circulaire.

4. Dispositif de fixation de partie d'os comme revendiqué dans une quelconque des revendications 1 à 4, dans lequel la au moins une caractéristique comprend une épaisseur et/ou largeur de la au moins une partie et de la au moins une autre partie.

5. Dispositif de fixation de partie d'os comme revendiqué dans une quelconque des revendications 1 à 4, dans lequel la partie d'expansion comprend au moins une partie allongée possédant une paire d'encoches allongées sur chaque côté de celle-ci.

6. Dispositif de fixation de partie d'os selon une quelconque des revendications 1 à 5, dans lequel la au moins une partie comprend une première extrémité de la au moins une partie allongée et une seconde extrémité de la au moins une partie allongée.

7. Dispositif de fixation de partie d'os comme revendiqué dans la revendication 6, dans lequel la au moins une autre partie comprend une partie médiane de la partie allongée formant le restant de la partie allongée.

8. Dispositif de fixation de partie d'os comme revendiqué dans l'une ou l'autre des revendications 6 ou 7, dans lequel la première extrémité et/ou la seconde extrémité de la partie allongée est plus mince ou plus épaisse et/ou plus étroite ou plus large que la partie adjacente de la au moins une partie allongée.

9. Dispositif de fixation de partie d'os comme revendiqué dans une quelconque des revendications 1 à 3, dans lequel la au moins une partie comprend ou comprend en outre une première extrémité (16a) de au moins une encoche et une seconde extrémité (16b) de au moins une encoche.

10. Dispositif de fixation de partie d'os comme revendiqué dans la revendication 9, dans lequel la au moins une autre partie comprend une partie médiane (18) de l'encoche formant le restant de l'encoche.

11. Dispositif de fixation de partie d'os comme revendiqué dans la revendication 1, dans lequel la première extrémité et/ou la seconde extrémité de la au moins une encoche est plus large que la partie adjacente de la au moins une encoche.

12. Dispositif de fixation d'os comme revendiqué dans une quelconque des revendications 1 à 3, dans lequel chaque partie d'expansion est mise à forme pour se plier élastiquement vers l'extérieur lorsqu'une force compressive est appliquée axialement aux parties d'expansion.

13. Dispositif de fixation d'os comme revendiqué dans une quelconque des revendications 1 à 3, dans lequel chaque partie d'expansion comprend au moins une partie longitudinale fixée en l'une ou l'autre extrémité à un moyen qui vient en prise avec un élément de couplage par compression (24), dans lequel le profil de la au moins une partie longitudinale se rétrécit en une ou en les deux extrémités de la au moins une partie longitudinale.

14. Dispositif de fixation de partie d'os comme revendiqué dans la revendication 13, dans lequel sont prévues plusieurs parties longitudinales espacées de manière essentiellement équidistante autour de la circonférence du module d'expansion.

15. Dispositif de fixation de partie d'os comme revendiqué dans l'une ou l'autre des revendications 13 ou 14, dans lequel chaque partie longitudinale possède un profil courbe ou en escalier.

16. Dispositif de fixation de partie d'os comme revendiqué dans une quelconque des revendications 13 à 15, dans lequel la surface extérieure de chaque partie longitudinale est dentelée (88) pour avoir prise sur la surface intérieure d'un os.

17. Dispositif de fixation de partie d'os comme revendiqué dans une quelconque des revendications 1 à 3, dans lequel chaque partie d'expansion comprend une encoche sur chaque côté longitudinal de celle-ci, chaque encoche possédant au moins une partie possédant une largeur plus grande que la largeur du restant de la au moins une encoche.

18. Dispositif de fixation de partie d'os comme revendiqué dans la revendication 17, dans lequel la au moins une partie de chaque encoche et le restant de chaque encoche sont déplacés longitudinalement.

19. Dispositif de fixation de partie d'os comme revendiqué dans l'une ou l'autre des revendications 17 ou 18, dans lequel la/chaque partie d'expansion comprend une pluralité d'encoches allongées.

20. Dispositif de fixation de partie d'os comme revendiqué dans une quelconque des revendications 17 à 19, dans lequel chaque encoche comprend des première et seconde parties plus larges en des première et seconde extrémités de l'encoche.

21. Dispositif de fixation de partie d'os comme revendiqué dans la revendication 20, dans lequel le restant de l'encoche est essentiellement de largeur uniforme.

22. Dispositif de fixation de partie d'os comme revendiqué dans la revendication 1, dans lequel le dispositif comprend une seule pièce de matériau tubulaire.

23. Dispositif de fixation de partie d'os comme revendiqué dans la revendication 1, dans lequel au moins une partie de la surface extérieure de chaque partie d'expansion est concave et au moins une partie de la surface intérieure de chaque partie d'expansion est concave.

24. Appareil de fixation de partie d'os comprenant:
au moins deux modules d'expansion, chaque module d'expansion comprenant un dispositif de fixation de partie d'os (10;10a;10b; 80; 90) selon une quelconque des revendications 1 à 23, chaque dispositif étant de confection unitaire essentiellement cylindrique, chaque partie d'expansion comprenant plusieurs parties essentiellement longitudinales qui, en service, sont essentiellement adjacentes à la paroi osseuse et qui plient élastiquement vers l'extérieur lorsqu'une force compressive est appliquée axialement au module d'expansion respectif;
au moins un élément de couplage par compression (24), l'élément de couplage par compression comprenant un moyen de fixation compressif pour venir en prise avec les modules d'expansion dans une position fixe par rapport à l'élément de couplage par compression et étant capable de transférer une force compressive; et
au moins un moyen de compression, le moyen de compression étant agencé pour transférer une force, telle qu'une force de rotation, appliquée à au moins une partie de la surface du moyen de compression, en service, en une force compressive appliquée à le au moins un élément de couplage par compression.

25. Appareil de fixation de partie d'os comme revendiqué dans la revendication 24, dans lequel plusieurs parties longitudinales sont espacées de manière essentiellement équidistantes autour de la circonférence de chaque module d'expansion et sont séparées l'une de l'autre par une encoche qui comporte une encoche allongée.

26. Appareil de fixation de partie d'os selon l'une ou l'autre des revendications 24 ou 25, dans lequel une ou les deux extrémités de chaque encoche sont arrondies dans la section transversale longitudinale.

27. Appareil de fixation de partie d'os comme revendiqué dans l'une ou l'autre des revendications 24 ou 25, dans lequel les parties longitudinales sont chargées en possédant un profil de surface courbe ou en escalier.

28. Appareil de fixation de partie d'os comme revendiqué dans la revendication 24, dans lequel le moyen de fixation compressif de l'élément de couplage par compression prend la forme d'une roue à rochet autobloquante.

29. Appareil de fixation de partie d'os comme revendiqué dans la revendication 24, dans lequel le moyen de fixation comprend au moins deux rainures anti-rotation (32a, 32b) qui viennent en prise avec une partie du module d'expansion de sorte que le module d'expansion ne puisse pas tourner par rapport à l'élément de couplage par compression.

30. Dispositif de fixation de partie d'os comme revendiqué dans une quelconque des revendications 1 à 23 ou appareil de fixation de partie d'os comme revendiqué dans une quelconque des revendications 25 à 29, dans lequel le dispositif ou le module d'expansion est constitué d'un matériau plastique à élasticité rigide, de titane ou d'un alliage de titane.

31. Appareil de fixation de partie d'os comme revendiqué dans la revendication 24, dans lequel l'appareil est adapté de sorte que, en service, un au moins un module d'expansion se trouve d'un côté d'une fracture dans un os et un autre au moins un module d'expansion se trouve d'un autre côté de la fracture,

32. Appareil de fixation de partie d'os comme revendiqué dans la revendication 24, dans lequel les modules d'expansion sont prévus sur une barre de liaison.

33. Appareil de fixation de partie d'os comme revendiqué dans la revendication 32, dans lequel les modules d'expansion adjacents sont couplés au moyen d'un élément de couplage par compression (24).
